# EUROPEAN PATENT APPLICATION

(11) **EP 1 955 654 A1**
(43) Date of publication of application: **13.08.2008**
(21) Application number: 06833475.4
(22) Date of filing: 28.11.2006
(51) Int. Cl.: A61B 5/151, A61B 5/15, A61M 5/32

(54) **BODY FLUID SAMPLER AND METHOD THEREFOR**

(30) Priority: 29.11.2005 JP 2005343377
(71) Applicant: TTM Co., Ltd., Osaka-shi, Osaka 532-0011 (JP)
(72) Inventor: USUI, Tsutomu, Osaka-shi, Osaka 532-0011 (JP)
(74) Representative: MacDougall, Alan John Shaw
(86) International application number: PCT/JP2006/323671
(87) International publication number: WO 2007/063828

(57) **Abstract**

To provide a body fluid sampling method and its device capable of sampling a minimum necessary amount of blood without loss while reducing a burden on a subject to the minimum. The skin surface of a sampling site is made water repellent. A skin surface thus made water repellent is pierced. A body fluid held in a droplet shape on the pierced skin surface is then collected.

## Description

### CROSS-REFERENCE TO RELATED APPLICATION

The entire disclosures of Japanese Patent Application No. 2005-343377 (filed November 29, 2005), inclusive of the specification, claims, drawings and abstract, are hereby incorporated by reference herein in their entireties.

### TECHNICAL FIELD

This invention relates to a device for sampling a body fluid such as blood and to a method therefor.

### BACKGROUND ART

Hitherto, in sampling a small amount of blood for testing the blood glucose level or the like, the skin of the subject has been pierced to allow blood to ooze. The blood is then directly or indirectly measured for the blood glucose level or the like.

For example, in the case of the direct measurement, a sensor portion of a measuring instrument is brought into contact with the site at which a predetermined amount of the blood is allowed to ooze. The blood is thus absorbed and supplied to the sensor for the measurement. In the case of the indirect measurement, the blood which is present on a fingertip, for example, is absorbed by an absorbent paper. The resulting absorbent paper is used for the measurement.

In the case where blood is allowed to ooze on the skin surface, there arises a problem that, a predetermined amount of the blood cannot be collected because, depending upon the amount of the blood and upon the angle of the skin surface, the blood to be sampled falls therefrom. For example, as shown in FIG. 99, the blood which has oozed by piercing a piercing site 91 of a fingertip 99, may spread from the pierced site and then flow down in the x-direction.

In this circumstance, a method is proposed in which a blood sampling adhesive tape provided with a through hole is used for preventing blood which has oozed on a skin surface from dropping (for example, Patent Document 1).

Patent Document 1: Japanese Unexamined Patent Application Publication No. 2005-74125(J P-A-2005-74125)

### DISCLOSURE OF THE INVENTION

### Problems to be Solved by the Invention:

Even when blood is sampled using the above-described blood sampling adhesive tape, however, it is apparent that the blood spreads throughout the range of the through hole. In such a case, it is not easy to absorb and supply a determined amount of the blood to the sensor of the measuring instrument without loss. Of course, it is possible to allow blood to ooze in a large amount by piercing deep into the skin surface and to absorb a determined amount of the blood. With such a method, however, a problem arises because a great burden such as pain and scars is imposed on the subject.

In particular, in SMBG (self monitoring of blood glucose), the technical improvement of the test instruments now enables to measure a blood glucose level with only a trace amount (about one micro-liter) of a blood sample. Therefore, when blood is not appropriately absorbed, blood must be allowed to ooze in excess of the necessary amount and, thus, the user is occasionally forced to accept an unwanted loss of blood.

The present invention has been made to solve the above-described problems and has as its object the provision of a body fluid sampling method and device capable of sampling a minimum necessary amount of blood without loss while reducing a burden on the subject to the minimum.

### Means for Solving the Problems:

(1) A body fluid sampling device according to the present invention is a body fluid sampling device for sampling a small amount of a body fluid, characterized by including an engaging surface configured to face a skin surface of a sampling site and to bear a water repellent agent, a lancet needle for piercing the skin surface which has been made water repellent by engagement with the engaging surface, and a collecting device for collecting a body fluid held in a droplet shape on the pierced skin surface.

As a result of the above constitution, it is possible to retain blood on the pierced skin surface and to collect a minimum necessary amount of blood without loss. Thus, it is possible to minimize the piercing depth and to reduce a burden on the subject to the minimum.

(2) A lancet according to the present invention is a lancet for sampling a small amount of a body fluid, characterized by including an engaging surface configured to face a skin surface of a sampling site and to bear a water repellent agent, and a lancet needle for piercing the skin surface which has been made water repellent by engagement with the engaging surface.

As a result of the above constitution, it is possible to hold blood on the pierced skin surface and to collect a minimum necessary amount of blood without loss. Thus, it is possible to minimize the piercing depth and to reduce a burden on the subject to the minimum.

(3) The body fluid sampling device or the lancet according to the present invention is characterized by further including a movable section movable together with the lancet needle.

As a result of the above constitution, it is possible to pierce the skin surface, which has been made water repellent, easily and surely by pushing the movable section.

(4) The body fluid sampling device or the lancet according to the present invention is characterized in that the lancet needle is configured to penetrate through the engaging surface.

As a result of the above constitution, it is possible to pierce the skin surface which has been made water repellent by engagement with the engaging surface. Thus, it is possible to maintain the blood on the skin surface in a spherical form, so that the collection thereof can be easily performed.

(5) The body fluid sampling device or the lancet according to the present invention is characterized in that the engaging surface is provided with a through hole near a center thereof, and that the lancet needle is configured to penetrate and pierce through the through hole.

As a result of the above constitution, it is possible to pierce the skin surface, which has been made water repellent by engagement with the engaging surface, with the lancet needle. Thus, it is possible to maintain the blood in a round cap-like form, so that the collection thereof can be easily performed.

(6) The body fluid sampling device or the lancet according to the present invention is characterized in that the engaging surface has a cotton material, a porous structure or an embossed structure for retaining the water repellent agent therein.

As a result of the above constitution, it is possible to surely make the skin surface water repellent by bringing the engaging surface into engagement therewith.

(7) A disinfectant alcohol according to the present invention is a disinfectant alcohol for disinfecting the skin from which a small amount of a body fluid is to be sampled, characterized by containing a water repellent agent for making the skin at a body fluid sampling site water repellent.

As a result of the above constitution, it is possible to perform both water repellency imparting operation and disinfection of the skin surface at the same time by disinfecting the skin surface with the disinfectant alcohol.

(8) A cotton material impregnated with a disinfectant alcohol according to the present invention is a cotton material for disinfecting the skin from which a small amount of a body fluid is to be sampled, characterized by containing a water repellent agent for making the skin at a body fluid sampling site water repellent.

As a result of the above constitution, it is possible to perform both water repellency imparting operation and disinfection of the skin surface at the same time by disinfecting the skin surface with the cotton material impregnated with a disinfectant alcohol.

(9) The body fluid sampling device, the lancet, the disinfectant alcohol or the cotton material impregnated with a disinfectant alcohol according to the present invention is characterized in that the water repellent agent includes silicone, fluorine or water repellent wax.

As a result of the above constitution, it is easy to retain the blood on the pierced skin surface. Namely, because the silicone, fluorine and water repellent wax are highly hydrophobic, it is possible to retain the blood in a spherical shape near the pierced portion.

(10) The body fluid sampling device, the lancet, the disinfectant alcohol or the cotton material impregnated with disinfectant alcohol according to the present invention is characterized in that the water repellent agent includes a solvent which is alcohol, emulsion liquid or oil.

As a result of the above constitution, when the alcohol is used, not only a water repellent effect but also an effect of sterilization and disinfection of the skin surface can be obtained at the same time.

Also, as a result of the above constitution, when the emulsion liquid or oil is used, not only a water repellent effect but also an effect of protection of the skin surface can be obtained at the same time.

(11) A body fluid sampling method according to the present invention is a body fluid sampling method for sampling a small amount of a body fluid, characterized by including a step of making a skin surface at a sampling site water repellent, a step of forming a pierced hole, using a lancet needle, at the skin surface which has been made water repellent, and a step of collecting a body fluid held in a droplet shape on the skin surface formed with the pierced hole.

As a result of the above constitution, it is possible to retain blood on the pierced skin surface and to collect a minimum necessary amount of blood without loss. Thus, it is possible to minimize the piercing depth and to reduce a burden on the subject to the minimum.

(12) The body fluid sampling method according to the present invention is characterized by further including a step of pushing a movable section movable together with the lancet needle, so that when the movable section is pushed after the skin surface has been made water repellent, the lancet needle pierces the skin surface to form the pierced hole.

As a result of the above constitution, it is possible to pierce the skin surface, which has been made water repellent, and to collect a body fluid easily and surely by pushing the movable section.

### Correspondence to Illustrated Embodiments:

(1) As used herein, the term "body fluid" is intended to mean a concept encompassing blood, plasma, lymph fluid, a tissue fluid and a cerebrospinal fluid that can be sampled through the skin. The term "sampling site" is intended to mean a concept including a determined area of the skin surface that includes a location at which a body fluid is sampled. The term "piercing the skin surface" is intended to mean a concept including puncturing the skin surface with a lancet needle from outside to inside the body. The term "water repellent agent" is intended to mean a concept including a liquid or solid having water repellent property such as a silicone, a fluorine and a water repellent wax. The term "to bear a water repellent agent" is intended to mean a concept including retaining the water repellent agent or a solution thereof in a cotton material or the like. The term "held in a droplet shape" is intended to mean a concept including maintaining body fluid in a bead-like form on a fingertip or the like.

(2) As used herein, the term "lancet" is intended to mean a concept including a skin puncturing instrument having a needle with a sharp tip.

(3) As used herein, the term "movable together with" is intended to mean a concept including a mechanism by which, when the movable section of the lancet is pushed, the lancet needle connected to the movable section moves together therewith.

(4) As used herein, the term "penetrate through the engaging surface" is intended to mean a concept including penetrating an extending area of the engaging surface having a center hole or penetrating the engaging surface having no center hole.

(5) As used herein, the term "provided with a through hole near a center thereof" is intended to mean a concept including a structure provided with a circular hole in the engaging surface for permitting the lancet needle to penetrate therethrough.

(6) As used herein, the term "a cotton material, a porous structure or an embossed structure for retaining the water repellent agent therein" is intended to refer to "a cotton material" which holds the water repellent agent by impregnation, "a porous structure" which holds the water repellent agent in respective pores by surface tension, or "an embossed structure" which holds the water repellent agent in interstices between projections and depressions formed on the surface.

(12) As used herein, the term "the movable section is pushed" is intended to refer to pushing the movable section toward the main body for outwardly extending the lancet needle retracted within the main body. The term "to form the pierced hole" is intended to refer to the formation of such a hole as to permit blood to ooze from capillaries in the subcutaneous tissue as a result of the piercing of the skin surface of the subject with the lancet needle.

Although the features of the present invention can be expressed as above in a broad sense, the constitution and content of the present invention, as well as the features and effects thereof, will be more apparent from the following disclosure, when considered in the light of the accompanying drawings.

### BRIEF DESCRIPTION OF THE DRAWINGS

FIG. 1 is a perspective view of a lancet according to the present invention.
FIG. 2 is views illustrating a piercing operation according to the present invention.
FIG. 3 is a view illustrating a fingertip after disinfection but before piercing according to the present invention.
FIG. 4 is a view illustrating a fingertip after the piercing according to the present invention.
FIG. 5 is views illustrating a piercing operation according to the present invention.
FIG. 6 is a view illustrating a fingertip after the piercing according to the present invention.
FIG. 7 is a view illustrating a body fluid sampling device according to the present invention.

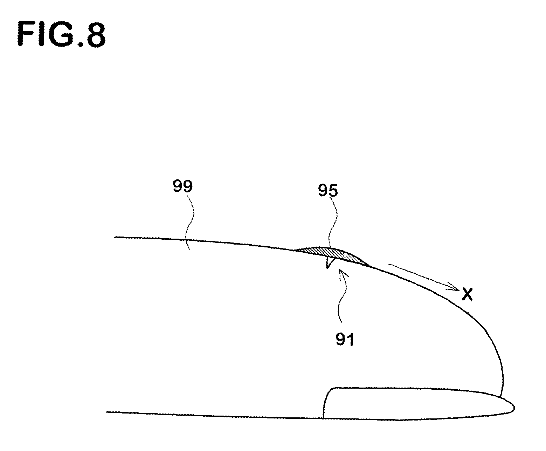

FIG. 8 is a view illustrating a fingertip after the piercing according to the present invention.
FIG. 9 is a perspective view of a lancet according to another embodiment of the present invention.
FIG. 10 is views illustrating a piercing operation according to another embodiment of the present invention.

### Explanation of Reference Numerals:

1: main body section
2: lancet needle
3: engaging surface
4: movable section

### BEST MODE FOR CARRYING OUT THE INVENTION

An embodiment of the present invention will be explained below with reference to the drawings. The embodiment, however, merely illustrates an example of the present invention. The size, material, pattern, configuration, etc. are not restricted to the illustrated embodiment but, rather, may be suitably modified and applied within a range not departing from the gist of the present invention.

### 1. First Embodiment:

### 1-1. Lancet

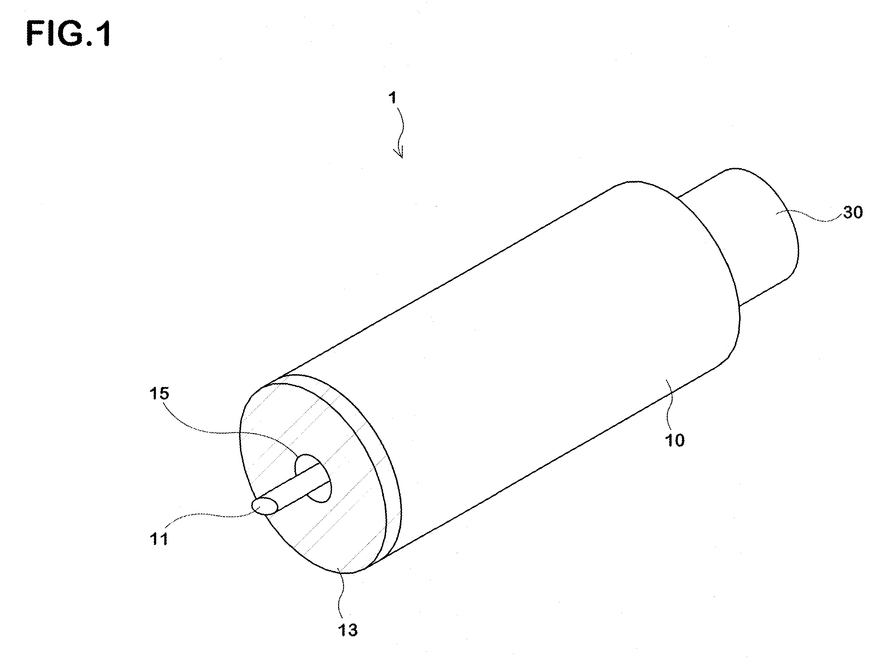

FIG. 1 is a perspective view of a lancet 1 according to the present invention. The lancet 1 is mainly composed of a main body section 10, a movable section 30, an engaging surface 13 and a lancet needle 11. The engaging surface 13 is constituted by attaching a cotton material to an end face of the main body section 10. The cotton material is impregnated with a disinfectant alcohol containing a water repellent agent dissolved therein. As the water repellent agent, there may be used a silicone, a fluorine or a water repellent wax. In place of the alcohol, an emulsion liquid or an oil may be used as a solvent.

In an initial state, the lancet needle 11 is retracted with the main body section 10. When the movable section 30 is pushed toward the main body section 10, the lancet needle 11, which is configured to be movable together with the movable section 30, outwardly extends from the main body 10 through a through hole 15 of the engaging surface 13.

### 1-2. Piercing

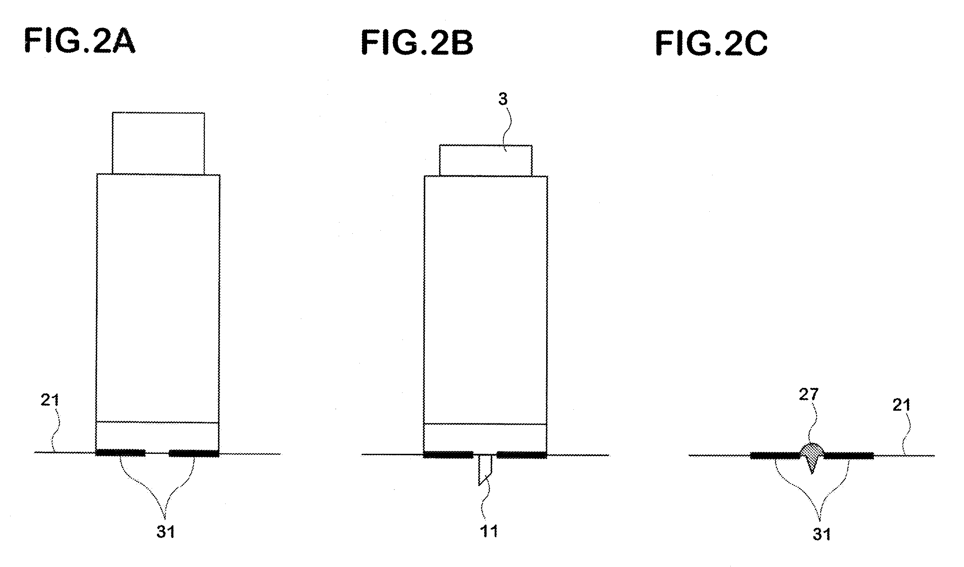

FIG. 2 are side views illustrating piercing operation using the lancet 1 shown in FIG. 1. FIG. 2A depicts a state where the engaging surface 13 of the lancet 1 is brought into contact with the skin surface 21. As described above, since the engaging surface 13 is constituted of the cotton material previously impregnated with the disinfectant alcohol containing the water repellent agent dissolved therein, a contacted area 25 of the skin surface 21 is made water repellent as a consequence of the engagement.

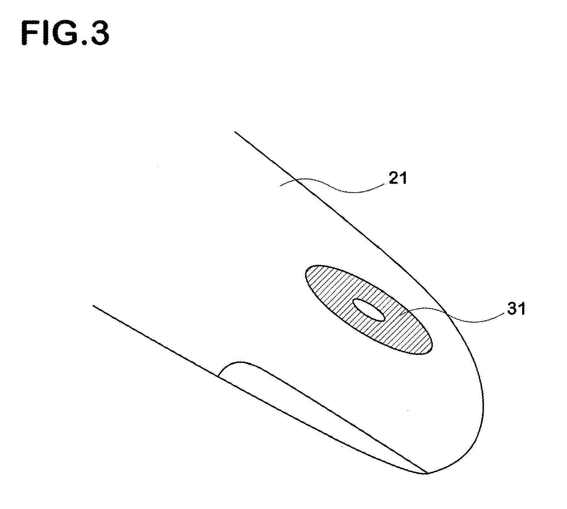

FIG. 3 depicts a state of the fingertip which is made water repellent by the lancet 1. The contacted area 31 of the skin surface 21 with which the engaging surface 13 has been brought into contact is made water repellent. Because of the presence of the through hole 15, the center region is not made water repellent.

In the state shown in FIG. 2A, when the movable section 30 of the lancet is pushed by the subject, the lancet needle 11 extends outward through the through hole 15 as shown in FIG. 2B. As a result, the skin surface 21 of the subject is pierced with the lancet needle 11.

When the lancet 1 is thereafter removed from the skin surface 21, a pierced hole is formed. Thus, blood begins oozing from the capillaries in the subcutaneous tissue broken with by the lancet needle 11 and the oozed blood 27 accumulates on the skin surface 21 as shown in FIG. 2C. In this case, since the contacted area 31 on the skin surface 21 has been made water repellent, the blood 27 is held in a round cap-like shape on the skin surface 21 as a result of the water repellent action of the contacted area 31.

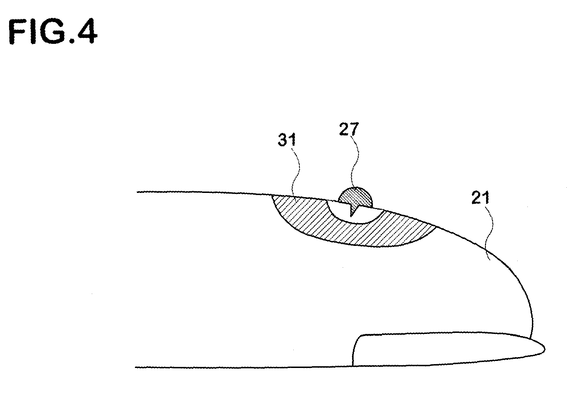

FIG. 4 depicts a state of a fingertip on which the blood 27 is held in a round cap-like shape on the skin surface 21 thereof. Because the contacted area 31 around the blood 27 has been made water repellent by the disinfectant alcohol containing the water repellent agent dissolved therein, the blood does not easily flow down even when the fingertip is slanted but, rather, is held in a round cap-like shape. In particular, because of the presence of the through hole 15 of the lancet 1, the region around the pierced portion is not made water repellent, and thus the round cap-like shape of the blood 27 can be stably retained. Thus, the blood 27 can be easily absorbed, particularly when a sensor etc. for measuring the blood glucose level is used.

### 1-3. Conclusion

With the lancet according to the present invention, because the skin surface is pierced after having been made water repellent, it is possible to retain blood in a round cap-like shape at a position near the pierced portion. Therefore, the blood is prevented from flowing down on the skin surface so that a minimum necessary amount of blood can be collected without loss. It follows that it is possible to minimize the piercing depth and the amount of the oozed blood and to reduce a burden on the subject to the minimum. Accordingly, it is only necessary to set the piercing depth and the size of the lancet needle in accordance with the minimum necessary amount of the blood sample.

### 2. Second Embodiment:

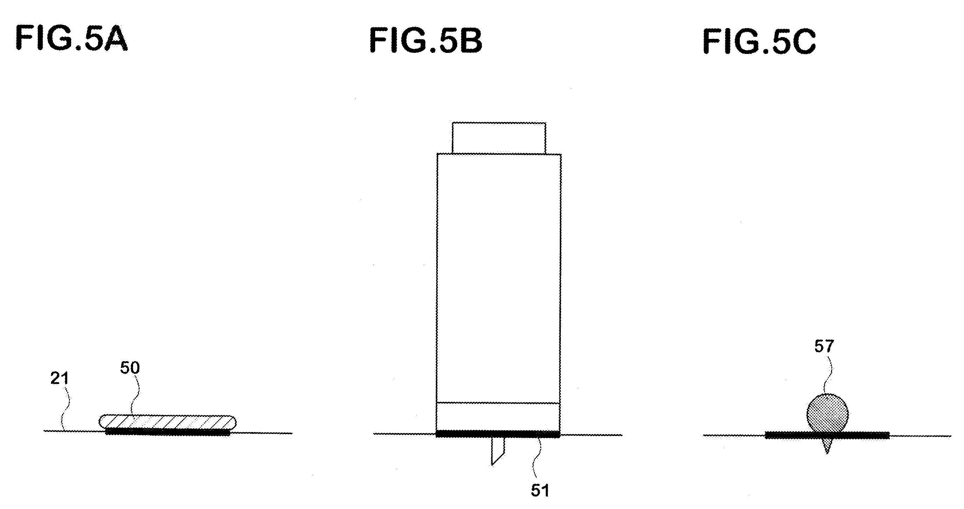

FIG. 5 are side views illustrating piercing method in which piercing is carried out using a conventional lancet after a skin surface 21 is made water repellent with a cotton material 50 impregnated with a disinfectant alcohol according to the present invention. In this case, the disinfectant alcohol-impregnated cotton material 50 individually packaged in an aluminum foil, etc may be used.

As shown in FIG. 5A, the subject wipes a portion to be pierced with the disinfectant alcohol-impregnated cotton material 50 to make a predetermined area 51 of the skin surface 21 water repellent. Then, as shown in FIG. 5B, a portion near the center of the predetermined area 51 which has been made water repellent is pierced using a conventional lancet. When the lancet is thereafter removed, blood accumulates near the pierced portion in the same manner as that descried previously. In this case, since the pierced portion has also been made water repellent, the blood 57 is held in a spherical form as shown in FIG. 5C.

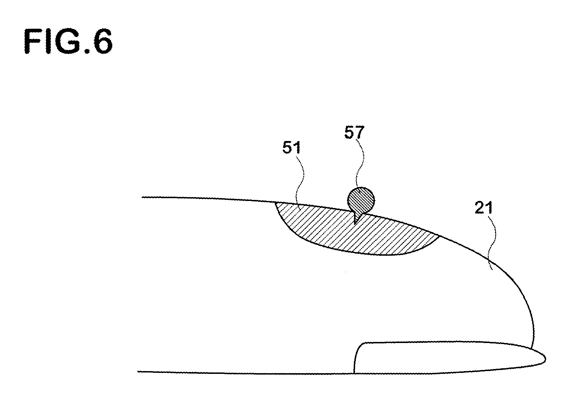

FIG. 6 depicts a state of a fingertip on which the blood 57 is retained in a spherical form on the skin surface 21 thereof. Because the pierced portion around the blood 57 has been made water repellent by the disinfectant alcohol containing the water repellent agent dissolved therein, the blood 57 is prevented from spreading around the skin surface 21 but, rather, is retained in a spherical form. Thus, the blood can be easily collected. In particular, when the blood is collected by impregnation in an absorbent paper, etc., the blood droplet can be easily fallen by turning down the fingertip because the blood has a spherical form.

### 3. Third Embodiment:

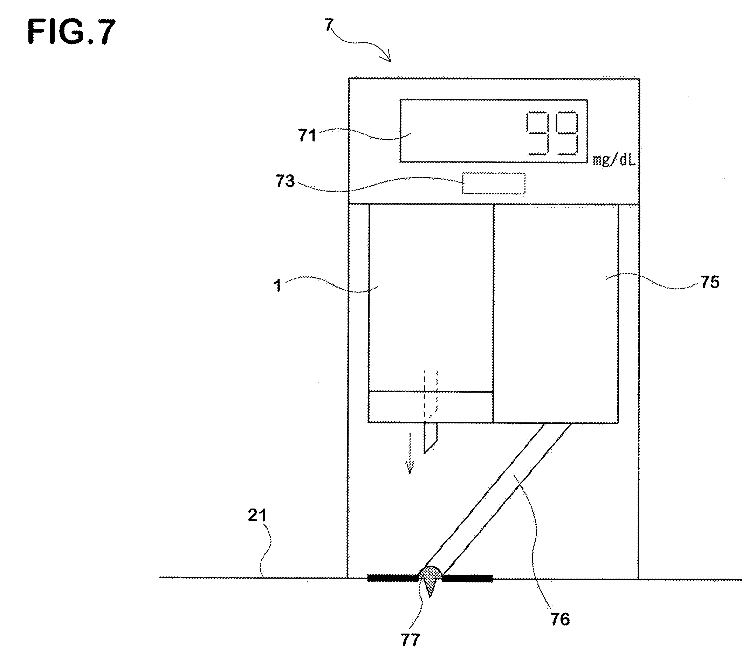

FIG. 7 is a view illustrating a body fluid sampling device 7 having the lancet 1 according to the present invention. The body fluid sampling device 7 is mainly composed of a display section 71, a sensor section 75 and the lancet 1. By using the body fluid sampling device 7, for example, it is possible to simultaneously perform the piercing with the lancet 1 and the measurement of blood glucose level with the sensor 8 and to display the results of the measurement in the display section 71.

When a measurement start button 73 is pushed after placement of the body fluid sampling device 7 on the skin surface 21, the lancet 1 displaces downward to make the skin surface 21 water repellent and to pierce the skin. After the piercing, the lancet 1 moves upward. Then an absorbing edge 76 of the sensor absorbs blood 77 which is held in a round cap-like shape on the pierced portion. Thus, measurement of the blood glucose level is started.

When the measurement of the blood glucose level is completed, the results of the measurement is indicated on the display 71.

Thus, the use of the lancet 1 according to the present invention permits absorbing of the blood 77, held in a round cap-like shape, by the sensor in a stable manner.

### 4. Fourth Embodiment

### 4-1. Lancet

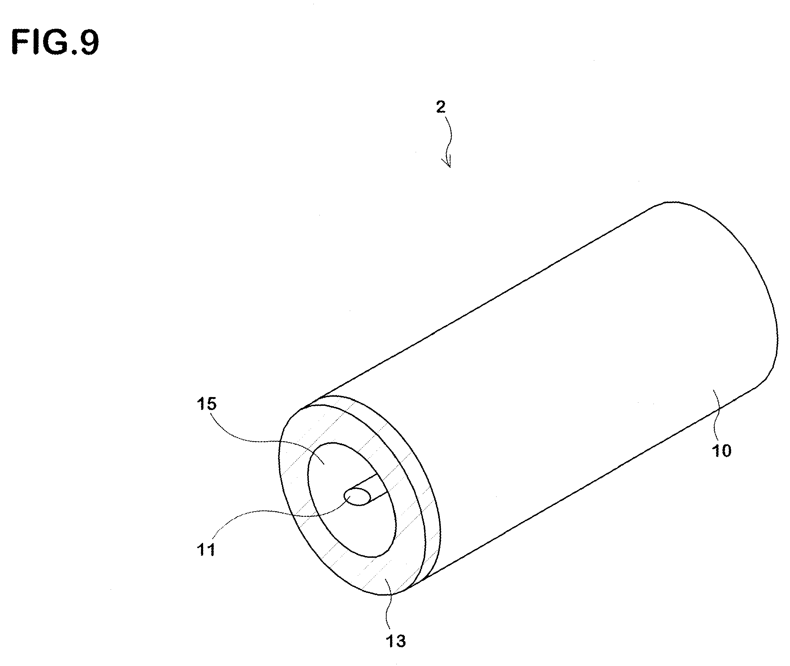

FIG. 9 is a perspective view of a lancet 2 according to another embodiment of the present invention. The lancet 2 is mainly composed of a main body section 10, an engaging surface 13 and a lancet needle 11. Similar to the first embodiment, the engaging surface 13 is constituted by attaching a cotton material to an end face of the main body section 10, with the cotton material being impregnated with a disinfectant alcohol containing a water repellent agent dissolved therein.

The lancet 2 of the this embodiment does not have such a movable section 30 as one in the lancet 1 of the first embodiment. In the lancet 2, there is formed a through hole 15 having a larger diameter than that in the lancet 1.

A lancet needle 11 is housed within the main body section 10. The housed position may be such that the tip end of the lancet needle 11 is located on a plane extending from the engaging surface 13, or such that the tip end of the lancet needle 11 is offset, either inside or outside, from a plane extending from the engaging surface 13. Since no movable section is provided, the lancet needle 11 does not move to extend outward.

In the above embodiment, the engaging surface 13 is constituted of the cotton material. However, the cotton material may be substituted with a material capable of holding a water repellent agent and having elasticity, such as a sponge. Such a structure has a merit that the lancet needle 11 can easily pierce the skin surface 21 which has been made water repellent.

### 4-2. Piercing

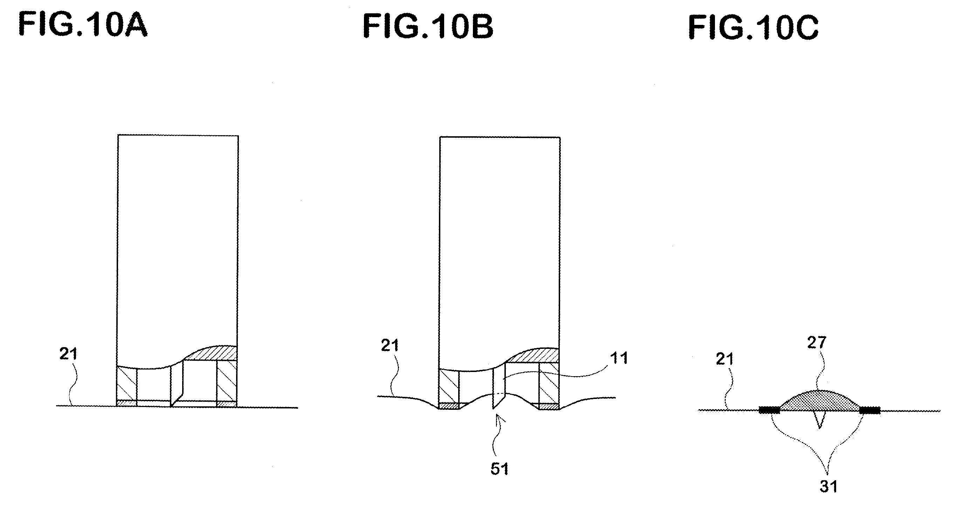

FIG. 10 are side views illustrating piercing operation using the lancet 2 shown in FIG. 9. FIG. 10Adepicts a state where the engaging surface 13 of the lancet 2 is brought into contact with the skin surface 21. As described above, since the engaging surface 13 is constituted of the cotton material previously impregnated with the disinfectant alcohol containing the water repellent agent dissolved therein, a contacted area 25 of the skin surface 21 is made water repellent as a consequence of the engagement.

As a result of the water repellency-imparting action by the lancet 2, the contacted area 31 of the skin surface 21 with which the engaging surface 13 has been brought into engagement is made water repellent. Because of the presence of a through hole 15, the center region is not made water repellent.

After the state shown in FIG. 10A has been established, the subject pushes the main body section 10 of the lancet 2 against the skin surface 21 in a direction normal to the skin surface 21. As a consequence, because of the presence of the through hole 15, a part of the skin surface 21 is raised to form a protrusion 210, as shown in FIG. 10B. Namely, the protrusion 210 is formed as a result of the positioning of the skin into the through hole 15.

In this case, because of the presence of the lancet needle 11 inside the through hole 15, the protrusion 210 is pierced with the lancet needle 11 when the main body section 10 is tightly pressed against the skin.

When the lancet 2 is removed from the skin surface 21, as shown in FIG. 10C, blood begins oozing from the capillaries in the subcutaneous tissue broken by the lancet needle 11, and the oozed blood 27 accumulates on the skin surface 21. In this case, since the contacted area 31 on the skin surface 21 has been made water repellent, the blood 27 is held in a round cap-like shape on the skin surface 21 as a result of the water repellent action of the contacted area 31.

Therefore, similar to the first embodiment, the blood 27 does not easily flow down but, rather, is held in a round cap-like shape.

### 5. Other Embodiments:

In the explanation of the above-described first embodiment, the piercing operation is performed using the lancet 1 having the engaging surface 13 provided with the through hole 15. However, the piercing can be carried out using a lancet having an engaging surface 13 which is not provided with such a through hole 15. In this case, when the movable section 30 is pushed toward the main body section 10, the lancet needle 11 which moves together therewith punctures the engaging surface 13 and extends outward from the main body section 10.

Thus, when the piercing is performed after the engaging surface 13 of the lancet 1 is brought into contact with the skin surface 21, the blood 27 is held in a round hat-like form on the skin surface as shown in FIG. 5C, because the skin surface 21 which has been brought into contact with the engaging surface 13 has been made water repellent.

In the explanation of the above-described embodiments, the engaging surface 13 of the lancet 1 is constituted of the cotton material previously impregnated with the disinfectant alcohol containing the water repellent agent dissolved therein. However, the engaging surface 13 may have any other constitution as long as it can retain the water repellent agent. For example, the engaging surface 13 may be formed to have a porous structure or an embossed structure and may be impregnated with the water repellent agent by being immersed in a solvent solution thereof.

In the explanation of the above-described embodiments, blood is sampled. However, these embodiments may be used to sample a body fluid other than blood, such as plasma, lymph fluid, a tissue fluid or a cerebrospinal fluid.

The above-described embodiments may be applied to any living organism as long as its skin surface can be made water repellent. For example, newts and fogs are illustrative of such organisms.

In the explanation of the above-described embodiments, the skin surface is made water repellent using a solvent (such as disinfectant alcohol, emulsion liquid or oil) in which the water repellent agent (such as silicone, fluorine or water repellent wax) is dissolved. In this case, the water repellent agent may be colored. For example, a coloring agent may be added to the water repellent agent or to the solvent.

While the summary of the present invention and the preferred embodiments of the present invention have been described in the foregoing, it should be understood that the terminology employed herein is for the purpose of illustration and not of restriction and that a person ordinary skilled in the art related to the present invention can understand and make other modifications of the method and device within the scope of the description of the present invention. Therefore, it should be understood that such modifications fall within the scope of the present invention.

## Claims

1. A body fluid sampling device for sampling a small amount of a body fluid, **characterized by** comprising:
an engaging surface configured to face a skin surface of a sampling site and to bear a water repellent agent;
a lancet needle for piercing the skin surface which has been made water repellent by engagement with the engaging surface; and
a collecting device for collecting a body fluid held in a droplet shape on the pierced skin surface.

2. A lancet for sampling a small amount of a body fluid, **characterized by** comprising:
an engaging surface configured to face a skin surface of a sampling site and to bear a water repellent agent; and
a lancet needle for piercing the skin surface which has been made water repellent by engagement with the engaging surface.

3. The body fluid sampling device or the lancet according to claim 1 or 2,
further comprising a movable section movable together with the lancet needle.

4. The body fluid sampling device or the lancet according to any one of claims 1 to 3,
wherein the lancet needle is configured to penetrate through the engaging surface.

5. The body fluid sampling device or the lancet according to claim 4,
wherein the engaging surface is provided with a through hole near a center thereof, and
wherein the lancet needle is configured to penetrate and pierce through the through hole.

6. The body fluid sampling device or the lancet according to any one of claims 1 to 5,
wherein the engaging surface has a cotton material, a porous structure or an embossed structure for retaining the water repellent agent therein.

7. A disinfectant alcohol for disinfecting the skin from which a small amount of a body fluid is sampled, **characterized by** containing a water repellent agent for making the skin at a body fluid sampling site water repellent.

8. The cotton material impregnated with a disinfectant alcohol for disinfecting the skin from which a small amount of a body fluid is to be sampled, **characterized by** containing a water repellent agent for making the skin at a body fluid sampling site water repellent.

9. The body fluid sampling device, the lancet, the disinfectant alcohol or the cotton material impregnated with the disinfectant alcohol according to any one of claims 1 to 8,
wherein the water repellent agent comprises silicone, fluorine or water repellent wax.

10. The body fluid sampling device, the lancet, the disinfectant alcohol or the cotton material impregnated with the disinfectant alcohol according to any one of claims 1 to 9,
wherein the water repellent agent comprises a solvent which is alcohol, emulsion liquid or oil.

11. A body fluid sampling method for sampling a small amount of a body fluid, **characterized by** comprising:
a step of making a skin surface at a sampling site water repellent;
a step of forming a pierced hole, using a lancet needle, at the skin surface which has been made water repellent; and
a step of collecting a body fluid held in a droplet shape on the skin surface formed with the pierced hole.

12. The body fluid sampling method according to claim 11, further comprising:
a step of pushing a movable section movable together with the lancet needle, so that when the movable section is pushed after the skin surface has is made water repellent, the lancet needle pierces the skin surface to form the pierced hole.
